# EUROPEAN PATENT SPECIFICATION

(11) **EP 2 561 864 B1**
(45) Date of publication and mention of the grant of the patent: **31.12.2014**
(21) Application number: 11460045.5
(22) Date of filing: 25.08.2011
(51) Int. Cl.: A61K 9/28, A61K 31/554, A61K 9/20

(54) **Coated tablet comprising tianeptine and process for preparation thereof**
Beschichtete Tablette mit Tianeptin und Verfahren zu deren Herstellung
Comprimés revêtus comportant de la tianeptine et leur procédé de préparation

(43) Date of publication of application: 27.02.2013
(73) Proprietor: Zaklady Farmaceutyczne Polpharma SA, 83-200 Starogard Gdanski (PL)
(72) Inventor: Stokrocka, Malgorzata, 80-180 Gdansk (PL)

(56) References cited:
- EP-A2- 0 004 516
- WO-A1-2010/051239
- US-A- 5 888 542
- BOIRET M ET AL: "Tablet potency of Tianeptine in coated tablets by near infrared spectroscopy: Model optimisation, calibration transfer and confidence intervals", JOURNAL OF PHARMACEUTICAL AND BIOMEDICAL ANALYSIS, NEW YORK, NY, US, vol. 54, no. 3, 20 February 2011 (2011-02-20), pages 510-516, XP027484311, ISSN: 0731-7085, DOI: 10.1016/J.JPBA.2010.09.029 [retrieved on 2010-10-01]

## Description

The invention relates to a coated tablet comprising tianeptine or pharmaceutically acceptable salt thereof as active ingredient, ethylcellulose present in the core of the tablet and at least one pharmaceutically acceptable excipient. The invention further relates to a process for the manufacture of a coated tablet comprising tianeptine or pharmaceutically acceptable salt thereof by blending tianeptine or pharmaceutically acceptable salt thereof and at least one pharmaceutically acceptable excipient, granulating thus obtained blend with a solution or suspension of ethylcellulose and compressing the granules to tablet cores.

Tianeptine is a drug having antidepressant activity. The compound was disclosed in British patent GB 1269551. A coated tablet comprising tianeptine sodium in the core of the tablet and coating comprising ethylcellulose is marketed by Les Laboratoires Servier under the trade name Coaxil. A film-coated tablet comprising tianeptine or pharmaceutically acceptable salt thereof present in the core of the tablet and film coating comprising ethylcellulose and a water-soluble polymer was disclosed in French patent FR 2881350.

Unexpectedly, it was found that a coated tablet comprising tianeptine or pharmaceutically acceptable salt thereof characterized by high uniformity of composition and high reproducibility of dissolution profile of the active ingredient from the tablet could be achieved by blending tianeptine or pharmaceutically acceptable salt thereof and at least one pharmaceutically acceptable excipient, granulating thus obtained blend with a solution or suspension of ethylcellulose, compressing the granules to tablet cores and coating the cores.

The invention relates to a coated tablet comprising tianeptine or pharmaceutically acceptable salt thereof as active ingredient, ethylcellulose present in the core of the tablet and at least one pharmaceutically acceptable excipient.

Tianeptine is preferably employed in the tablet of the present invention in the form of sodium salt in an amount of 1 to 20% by total weight of the coated tablet.

In a preferred embodiment of the invention, the core of the tablet is formed of granules comprising ethylcellulose, at least one pharmaceutically acceptable excipient and tianeptine.

In another preferred embodiment of the invention, ethylcellulose is present in the core of the tablet in an amount of 0.2 to 10% by total weight of the coated tablet, more preferably in an amount of 0.3 to 6%, even more preferably in an amount of 0.5 to 4%, most preferably in an amount of 0.8 to 2.5% by total weight of the coated tablet.

In yet another preferred embodiment of the invention, the pharmaceutically acceptable excipient is a disintegrant. The disintegrant according to the present invention is preferably employed in the tablet in an amount of 2 to 10% by total weight of the coated tablet and is preferably selected from the group comprising crospovidone, croscarmellose, sodium starch glycolate, maize starch and mixtures thereof.

In another preferred embodiment of the invention, the pharmaceutically acceptable excipient present in the core of the tablet further comprises at least one filler and/or binder. The term "binder" is to be understood in the present invention as a pharmaceutically acceptable excipient used to impart cohesiveness to the tablet formulation in order to assure that tablet remains intact after compression and not being ethylcellulose.

The binder and/or filler according to the present invention is preferably employed in the tablet in an amount of 10 to 90% by total weight of the coated tablet. Some fillers and binders can be used interchangeably as they may exhibit both properties. The binder is preferably selected from the group comprising povidone, hydroxypropyl methylcellulose, hydroxyethylcellulose and mixtures thereof. The filler is preferably selected from the group comprising lactose, mannitol, microcrystalline cellulose, dibasic calcium phosphate and mixtures thereof.

In a preferred embodiment of the invention, the core of the tablet further comprises at least one glidant and/or lubricant. Some glidants and lubricants can be used interchangeably as they may exhibit both properties. The glidant is preferably selected from the group comprising colloidal silicon dioxide, calcium silicate, magnesium trisilicate and mixtures thereof. The lubricant is preferably selected from the group comprising sodium stearyl fumarate, talc, magnesium stearate, calcium stearate and zinc stearate and mixtures thereof.

In another preferred embodiment of the invention, coating of the tablet comprises polyvinyl alcohol.

The invention relates also to a process for preparation of a coated tablet comprising tianeptine or pharmaceutically acceptable salt thereof as active ingredient. The process of the present invention comprises blending tianeptine or pharmaceutically acceptable salt thereof with at least one pharmaceutically acceptable excipient, forming granules by granulating thus obtained blend with a solution or suspension of ethylcellulose and compressing the granules to tablet cores.

In a preferred embodiment of the invention, ethylcellulose is employed in an amount of 0.2 to 10% by total weight of the tablet, more preferably in an amount of 0.3 to 6%, even more preferably in an amount of 0,5 to 4%, most preferably in an amount of 0.8 to 2.5% by total weight of the tablet.

In another preferred embodiment of the invention, the pharmaceutically acceptable excipient blended with tianeptine is a disintegrant.

In yet another preferred embodiment of the invention, the pharmaceutically acceptable excipient blended with tianeptine further comprises at least one filler and/or binder.

In another preferred embodiment of the invention, the process further comprises adding at least one glidant and/or lubricant to the granules before compressing to tablet cores.

In yet another preferred embodiment of the invention, the tablet cores are further coated with a coating suspension.

In another preferred embodiment of the invention, the coating suspension comprises polyvinyl alcohol.

The following examples illustrate various aspects of the present invention.

### Example 1

Composition of a coated tablet comprising tianeptine sodium and manufacturing process for a batch size 9.2 kg.

| **Ingredient** | **Amount [mg/tabl.]** | **Amount per batch [g]** |
|---|---|---|
| ***Core*** | | |
| Tianeptine sodium | 12.5 | 697.5 |
| Mannitol | 135.0 | 7,531.8 |
| Talc | 6.8 | 379.4 |
| Maize starch | 6.0 | 334.7 |
| Ethylcellulose | 2.2 | 122.7 |
| Magnesium stearate | 2.4 | 133.9 |
| **Total mass of core** | **164.9** | **9,200.0** |

| ***Coating*** | | |
|---|---|---|
| Polyvinyl alcohol | 8.0 | 446.3 |
| Titanium dioxide | 4.92 | 274.5 |
| PEG 3350 | 4.04 | 225.5 |
| Quinoline yellow aluminium lake | 0.072 | 4.0 |
| Ponceau 4R lake | 0.008 | 0.5 |
| **Total mass of coating excipients** | **20.0** | **1,115.8** |
| **Total mass of coated tablet** | **184.9** | **10,315.8** |

Tianeptine sodium, mannitol, talc and maize starch in above prescribed amounts were blended and wet granulated with a solution of ethylcellulose in ethanol. Thus obtained granulate was blended with magnesium stearate and compressed to tablet cores using rotary tablet press. Tablet cores of total weight of 164.9 mg were obtained.

Polyvinyl alcohol, titanium dioxide, PEG 3350, quinoline yellow aluminium lake and ponceau 4R lake in above prescribed amounts (commercially available from Colorcon under trade name Opadry) are dispersed in purified water. Tablet cores are placed in a coating pan and sprayed with thus obtained coating suspension. Coated tablets of total weight of 184.9 mg were obtained.

### Example 2

Composition of a coated tablet comprising tianeptine sodium and manufacturing process for a batch size 9.2 kg.

| **Ingredient** | **Amount [mg/tabl.]** | **Amount per batch [g]** |
|---|---|---|
| ***Core*** | | |
| Tianeptine sodium | 12.5 | 697.5 |
| Lactose | 135.0 | 7,531.8 |
| Talc | 6.8 | 379.4 |
| Crospovidone | 6.0 | 334.7 |
| Ethylcellulose | 2.2 | 122.7 |
| Magnesium stearate | 2.4 | 133.9 |
| **Total mass of core** | **164.9** | **9,200.0** |

| ***Coating*** | | |
|---|---|---|
| Polyvinyl alcohol | 8.0 | 446.3 |
| Titanium dioxide | 4.92 | 274.5 |
| PEG 3350 | 4.04 | 225.5 |
| Quinoline yellow aluminium lake | 0.072 | 4.0 |
| Ponceau 4R lake | 0.008 | 0.5 |
| **Total mass of coating excipients** | **20.0** | **1,115.8** |
| **Total mass of coated tablet** | **184.9** | **10,315.8** |

Manufacturing process analogous to that of example 1. Coated tablets of total weight of 184.9 mg are obtained.

### Example 3

Composition of a coated tablet comprising tianeptine sodium and manufacturing process for a batch size 9.2 kg.

| **Ingredient** | **Amount [mg/tabl.]** | **Amount per batch [g]** |
|---|---|---|
| ***Core*** | | |
| Tianeptine sodium | 12.5 | 697.5 |
| Microcrystalline cellulose | 135.0 | 7,531.8 |
| Talc | 6.8 | 379.4 |
| Croscarmellose | 6.0 | 334.7 |
| Ethylcellulose | 2.2 | 122.7 |
| Magnesium stearate | 2.4 | 133.9 |
| **Total mass of core** | **164.9** | **9,200.0** |

| ***Coating*** | | |
|---|---|---|
| Polyvinyl alcohol | 8.0 | 446.3 |
| Titanium dioxide | 4.92 | 274.5 |
| PEG 3350 | 4.04 | 225.5 |
| Quinoline yellow aluminium lake | 0.072 | 4.0 |
| Ponceau 4R lake | 0.008 | 0.5 |
| **Total mass of coating excipients** | **20.0** | **1,115.8** |
| **Total mass of coated tablet** | **184.9** | **10,315.8** |

Manufacturing process analogous to that of example 1. Coated tablets of total weight of 184.9 mg are obtained.

### Comparative example

### Composition of a tablet comprising tianeptine sodium and no ethylcellulose in the core of the tablet coated with a solution of ethylcellulose in ethanol.

| **Ingredient** | **Amount [mg/tabl.]** | **Amount per batch [g]** |
|---|---|---|
| ***Core*** | | |
| Tianeptine sodium | 12.5 | 125.0 |
| Mannitol | 135.0 | 1,350.0 |
| Talc | 5.0 | 50.0 |
| Maize starch | 5.1 | 51.0 |
| Magnesium stearate | 2.4 | 24.0 |
| **Total mass of core** | **160.0** | **1,600.0** |

| ***Coating*** | | |
|---|---|---|
| Ethylcellulose | 0.8 | 8.0 |
| **Total mass of coating excipients** | **0.8** | **8.0** |
| **Total mass of coated tablet** | **160.8** | **1,608.0** |

Tianeptine sodium, mannitol, talc and maize starch in above prescribed amounts were blended. Thus obtained mixture was blended with magnesium stearate and compressed to tablet cores using rotary tablet press. Tablet cores of total weight of 160.0 mg were obtained.

Ethylcellulose in above prescribed amount was dissolved in ethanol. Tablet cores were coated with thus obtained solution. Coated tablets of total weight of 160.8 mg were obtained.

Dissolution profiles of tianeptine from coated tablets of example 1 and comparative example were tested after 10, 15, 30, 60 and 90 minutes in 0.1 N HCl at 37°C±0.5°C using basket apparatus at 100 RPM. The following results were obtained:

| | **Tablet of example 1** | | **Tablet of comparative example** | |
|---|---|---|---|---|
| Time (min.) | Av. release (6 tabl.) | RSD* | Av. release (6 tabl.) | RSD* |
| 10 | 31.5 | **0.9** | 4.8 | **75.8** |
| 15 | 38.5 | **1.9** | 8.8 | **92.0** |
| 30 | 73.9 | **2.1** | 33.1 | **76.3** |
| 60 | 96.0 | **2.3** | 92.7 | **22.9** |
| 90 | 100.1 | **0.8** | 104.8 | **9.4** |

| | | | | |
|---|---|---|---|---|
| *RSD - Relative standard deviation | | | | |

Low values of RSD of release of tianeptine from coated tablets of example 1 prove high repeatability of dissolution profile of the active ingredient from the tablet of the present invention. In contrast, tablets of the comparative example obtained according to the methods known to the skilled person and coated with ethylcellulose show much higher values of RSD of release, i.e. much lower repeatability of dissolution profile of tianeptine from the tablet.

## Claims

1. A coated tablet comprising tianeptine or pharmaceutically acceptable salt thereof as active ingredient, ethylcellulose and at least one pharmaceutically acceptable excipient, **characterized in that** ethylcellulose is present in the core of the tablet.

2. The tablet according to claim 1, wherein the core of the tablet is formed of granules comprising ethylcellulose, at least one pharmaceutically acceptable excipient and tianeptine.

3. The tablet according to claims 1 or 2, wherein ethylcellulose is present in the core of the tablet in an amount of 0.2 to 10% by total weight of the coated tablet, more preferably in an amount of 0.3 to 6%, even more preferably in an amount of 0.5 to 4%, most preferably in an amount of 0.8 to 2.5% by total weight of the coated tablet.

4. The tablet according to any preceding claim, wherein the pharmaceutically acceptable excipient is a disintegrant.

5. The tablet according to any preceding claim, wherein the pharmaceutically acceptable excipient further comprises at least one filler and/or binder.

6. The tablet according to any preceding claim, wherein the core of the tablet further comprises at least one glidant and/or lubricant.

7. The tablet according to any preceding claim, wherein coating of the tablet comprises polyvinyl alcohol.

8. A process for preparation of a coated tablet comprising tianeptine or pharmaceutically acceptable salt thereof as active ingredient, **characterized in that** the process comprises blending tianeptine or pharmaceutically acceptable salt thereof with at least one pharmaceutically acceptable excipient, forming granules by granulating thus obtained blend with a solution or suspension of ethylcellulose and compressing the granules to tablet cores.

9. The process according to claim 8, wherein ethylcellulose is employed in an amount of 0.2 to 10% by total weight of the coated tablet, more preferably in an amount of 0.3 to 6%, even more preferably in an amount of 0.5 to 4%, most preferably in an amount of 0.8 to 2.5% by total weight of the coated tablet.

10. The process according to any of claims 8 or 9, wherein the pharmaceutically acceptable excipient is a disintegrant.

11. The process according to any of claims 8 to 10, wherein the pharmaceutically acceptable excipient further comprises at least one filler and/or binder.

12. The process according to any of claims 8 to 11, wherein the process further comprises adding at least one glidant and/or lubricant to the granules.

13. The process according to any of claims 8 to 12, wherein the tablet cores are further coated with a coating suspension.

14. The process according to claim 13, wherein the coating suspension comprises polyvinyl alcohol.

## Patentansprüche

1. Beschichtete Tablette, umfassend Tianeptin oder ein pharmazeutisch unbedenkliches Salz davon als Wirkstoff, Ethylcellulose und mindestens einen pharmazeutisch unbedenklichen Hilfsstoff, **dadurch gekennzeichnet, dass** Ethylcellulose im Kern der, Tablette vorliegt.

2. Tablette nach Anspruch 1, wobei der Kern der Tablette aus einem Ethylcellulose, mindestens einem pharmazeutisch unbedenklichen Hilfsstoff und Tianeptin umfassenden Granulat gebildet ist.

3. Tablette nach Anspruch 1 oder 2, wobei Ethylcellulose im Kern der Tablette in einer Menge von 0,2 bis 10%, bezogen auf das Gesamtgewicht der beschichteten Tablette, weiter bevorzugt in einer Menge von 0,3 bis 6%, noch weiter bevorzugt in einer Menge von 0,5 bis 4% und besonders bevorzugt in einer Menge von 0,8 bis 2,5%, bezogen auf das Gesamtgewicht der beschichteten Tablette, vorliegt.

4. Tablette nach einem der vorhergehenden Ansprüche, bei dem es sich bei dem pharmazeutisch unbedenklichen Hilfsstoff um ein Zerfallsmittel handelt.

5. Tablette nach einem der vorhergehenden Ansprüche, wobei der pharmazeutisch unbedenkliche Hilfsstoff ferner mindestens einen Füllstoff und/oder mindestens ein Bindemittel umfasst.

6. Tablette nach einem der vorhergehenden Ansprüche, wobei der Kern der Tablette ferner mindestens ein Gleitmittel und/oder Schmiermittel umfasst.

7. Tablette nach einem der vorhergehenden Ansprüche, wobei die Beschichtung der Tablette Polyvinyl-alkohol umfasst.

8. Verfahren zur Herstellung einer beschichteten Tablette, die Tianeptin oder ein pharmazeutisch unbedenkliches Salz davon als Wirkstoff enthält, **dadurch gekennzeichnet, dass** man dabei Tianeptin oder ein pharmazeutisch unbedenkliches Salz davon mit mindestens einem pharmazeutisch unbedenklichen Hilfsstoff mischt, durch Granulieren der so erhaltenen Mischung mit einer Lösung oder Suspension von Ethylcellulose ein Granulat bildet und das Granulat zu Tablettenkernen verpresst.

9. Verfahren nach Anspruch 8, bei dem man Ethylcellulose in einer Menge von 0,2 bis 10%, bezogen auf das Gesamtgewicht der beschichteten Tablette, weiter bevorzugt in einer Menge von 0,3 bis 6%, noch weiter bevorzugt in einer Menge von 0,5 bis 4% und besonders bevorzugt in einer Menge von 0,8 bis 2,5%, bezogen auf das Gesamtgewicht der beschichteten Tablette, einsetzt.

10. Verfahren nach Anspruch 8 oder 9, bei dem es sich bei dem pharmazeutisch unbedenklichen Hilfsstoff um ein Zerfallsmittel handelt.

11. Verfahren nach einem der Ansprüche 8 bis 10, bei dem der pharmazeutisch unbedenkliche Hilfsstoff ferner mindestens einen Füllstoff und/oder mindestens ein Bindemittel umfasst.

12. Verfahren nach einem der Ansprüche 8 bis 11, bei dem man ferner dem Granulat mindestens ein Gleitmittel und/oder Schmiermittel zusetzt.

13. Verfahren nach einem der Ansprüche 8 bis 12, bei dem man die Tablettenkerne ferner mit einer Beschichtungssuspension beschichtet.

14. Verfahren nach Anspruch 13, bei dem die Beschichtungssuspension Polyvinylalkohol umfasst.

## Revendications

1. Comprimé enrobé comprenant de la tianeptine ou un sel pharmaceutiquement acceptable de celle-ci en tant que principe actif, de l'éthylcellulose et au moins un excipient pharmaceutiquement acceptable, **caractérisé en ce que** l'éthylcellulose est présente dans le noyau du comprimé.

2. Comprimé selon la revendication 1, le noyau du comprimé étant constitué de granulés comprenant de l'éthylcellulose, au moins un excipient pharmaceutiquement acceptable et de la tianeptine.

3. Comprimé selon les revendications 1 ou 2, l'éthylcellulose étant présente dans le noyau du comprimé en une quantité de 0,2 à 10 % en poids par rapport au poids total du comprimé enrobé, de préférence encore en une quantité de 0,3 à 6 %, de préférence même encore en une quantité de 0,5 à 4 %, de préférence par-dessus tout en une quantité de 0,8 à 2,5 % en poids par rapport au poids total du comprimé enrobé.

4. Comprimé selon l'une quelconque des revendications précédentes, dans lequel l'excipient pharmaceutiquement acceptable est un délitant.

5. Comprimé selon l'une quelconque des revendications précédentes, dans lequel l'excipient pharmaceutiquement acceptable comprend en outre au moins une charge et/ou un liant.

6. Comprimé selon l'une quelconque des revendications précédentes, le noyau du comprimé comprenant en outre au moins un agent de glissement et/ou lubrifiant.

7. Comprimé selon l'une quelconque des revendications précédentes, l'enrobage du comprimé comprenant du poly(alcool vinylique).

8. Procédé pour la préparation d'un comprimé enrobé comprenant de la tianeptine ou un sel pharmaceutiquement acceptable de celle-ci en tant que principe actif, **caractérisé en ce que** le procédé comprend le mélange de tianeptine ou d'un sel pharmaceutiquement acceptable de celle-ci avec au moins un excipient pharmaceutiquement acceptable, la formation de granulés par granulation du mélange ainsi obtenu avec une solution ou suspension d'éthylcellulose et la compression des granulés en noyaux de comprimés.

9. Procédé selon la revendication 8, dans lequel l'éthylcellulose est employée en une quantité de 0,2 à 10 % en poids par rapport au poids total du comprimé enrobé, de préférence encore en une quantité de 0,3 à 6 %, de préférence même encore en une quantité de 0,5 à 4 %, de préférence par-dessus tout en une quantité de 0,8 à 2,5 % en poids par rapport au poids total du comprimé enrobé.

10. Procédé selon l'une quelconque des revendications 8 ou 9, dans lequel l'excipient pharmaceutiquement acceptable est un délitant.

11. Procédé selon l'une quelconque des revendications 8 à 10, dans lequel l'excipient pharmaceutiquement acceptable comprend en outre au moins une charge et/ou un liant.

12. Procédé selon l'une quelconque des revendications 8 à 11, le procédé comprenant en outre l'ajout d'au moins un agent de glissement et/ou lubrifiant aux granulés.

13. Procédé selon l'une quelconque des revendications 8 à 12, dans lequel les noyaux de comprimés sont en outre enrobés avec une suspension d'enrobage.

14. Procédé selon la revendication 13, dans lequel la suspension d'enrobage comprend du poly(alcool vinylique).
